# EUROPEAN PATENT SPECIFICATION

(11) **EP 2 029 057 B1**
(45) Date of publication and mention of the grant of the patent: **18.03.2015**
(21) Application number: 07825802.7
(22) Date of filing: 07.06.2007
(51) Int. Cl.: A61F 2/24

(54) **DEVICE FOR REPLACING THE CHORDAE TENDINEAE OF AN ATRIOVENTRICULAR VALVE**
VORRICHTUNG ZUM AUSTAUSCHEN DER SEHNENFÄDEN EINER ATRIOVENTRIKULÄREN KLAPPE
DISPOSITIF POUR REMPLACER LES CORDES TENDINEUSES D'UNE VALVE ATRIOVENTRICULAIRE

(30) Priority: 07.06.2006 IT TO20060413
(43) Date of publication of application: 04.03.2009
(73) Proprietor: Scorsin, Marcio, 10125 Torino (IT)
(72) Inventor: Lessana, Arrigo, 7500 Paris (FR); Scorsin, Marcio, 10125 Torino (IT)
(74) Representative: Vanzini, Christian
(86) International application number: PCT/IB2007/052162
(87) International publication number: WO 2008/007243

(56) References cited:
- WO-A-96/40007
- US-A- 5 415 667
- US-A1- 2004 143 323

## Description

The present invention relates to a device for replacing the chordae tendineae of an atrioventricular valve.

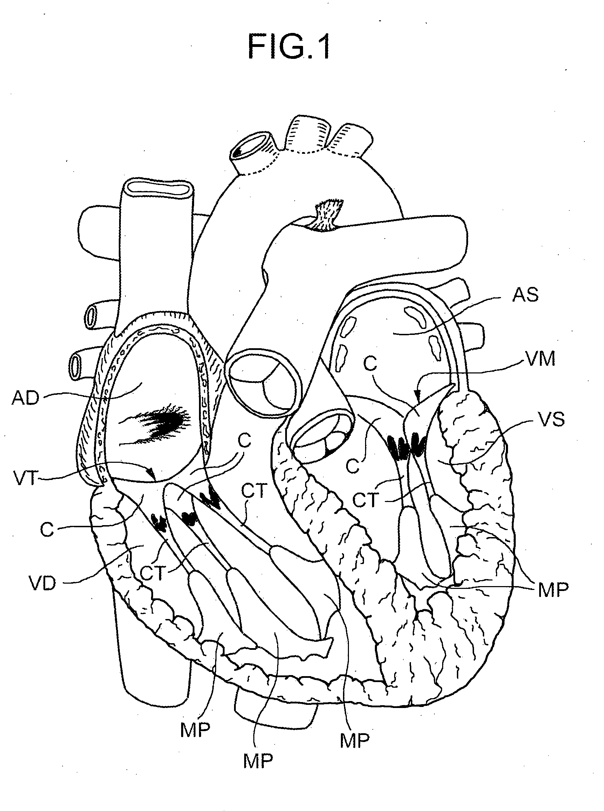

As is known, inside the heart atrioventricular communication occurs by means of atrioventricular valves, which are essentially non-return valves. With reference to Figure 1, the valve VT arranged at the atrioventricular orifice which connects the right atrium AD to the right ventricle VD is called the tricuspid valve, and the valve VM arranged at the atrioventricular orifice which connects the left atrium AS to the left ventricle VS is called the mitral valve. The tricuspid valve VT is formed by three leaflets or cusps C, and the mitral valve VM is formed by two leaflets or cusps C. Each of the atrioventricular valves VT and VM comprises a ring (not visible) consisting of a fibrous tissue to which the bases of the cusps C are connected. Also present are muscular tissue parts MP, the so-called papillary muscles, which project from the wall of the respective ventricle VS, VD. From the end of each of the cusps C there extends a bundle of taut fibres CT, the so-called chordae tendineae, which are attached to the papillary muscles MP. Activation of the valves occurs passively. The tension produced in the chordae tendineae CT by the contraction of the papillary muscle MP at the start of the systole balances the increase in the intraventricular pressure which would tend to cause the valve to open inwards, giving rise to a backflow towards the atrium.

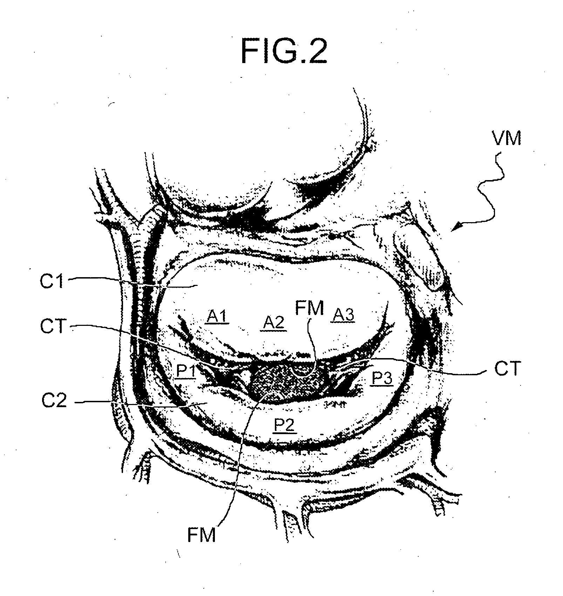

With reference in particular to Figure 2, the mitral valve complex VM comprises the mitral annulus (not visible), the anterior cusp C1 and the posterior cusp C2, the chordae tendineae CT and the papillary muscles MP (visible in Fig. 1). The mitral annulus forms the base of the mitral cusps C1 e C2 and is an elliptical ring which undergoes variations during the heart cycle, changing in size and being subject to three-dimensional movements.

When the valve is closed, the two cusps, i.e. posterior cusp C2 and anterior cusp C1, have substantially the same area. The area of the surface of both the cusps C1, C2 is equal to about twice the area of the mitral orifice. This extra large surface area of the cusps ensures that the orifice is covered under normal conditions and allows for compensation in pathological cases. The anterior cusp C1 is slightly bigger than the posterior cusp C2 and has a semi-circular or triangular shape. The posterior cusp C2 has an undulating shape and is generally divided into three or more parts which are referred to as "scallops". These scallops are generally indicated by P1, P2 and P3. Generally, also the anterior cusp C1 is divided into three parts, denoted by A1, A2 and A3, corresponding to the scallops of the posterior cusp C2. Close to the free edge FM of the cusps C1 and C2 the atrial surface of these cusps is irregular with nodular thickened parts. This zone is generally called rough zone.

Part of the chordae tendineae is attached at one end to the ventricular side of the rough zone, or in any case close to the free edge of the cusp, corresponding to the closing line of the mitral valve VM, and at the other end to the tip of the two papillary muscles. When viewing the closed valve in profile, most of the closing line is situated underneath the plane of the atrioventricular junction, so that the atrial surface of the cusps has a saddle-like configuration. The area of the orifice of the mitral valve at the level of the mitral annulus is approximately 6.5 cm² for women and 8 cm² for men. The circumference of the mitral annulus is approximately 9 cm for women and 10 cm for men.

Among the pathologies affecting the mitral valve there is the so-called prolapse. By definition, prolapse of the mitral valve is the situation where the cusps extend above the plane of the atrioventricular junction during the ventricular systole. The most common form of prolapse occurs when the free edge of the affected cusp passes beyond the closing line of the opposite cusp with inevitable regurgitation. One of the causes of prolapse is breakage of the chordae tendineae.

In past years surgeons have treated prolapsed valves by means of resection of the prolapsed segment (mainly for prolapse of the posterior cusp) or, in the case where the prolapse is situated on the anterior cusp, by means of partial transposition of a segment of the posterior cusp with intact chords into the corresponding damaged anterior cusp. However, mitral plasty techniques require a great deal of experience and have a long learning curve. Another important aspect consists in the fact that the affected part in this type of mitral prolapse is not the cusp, but the cords, and only the latter need be repaired. Artificial cords have been used for this purpose with good results; however, a considerable amount of controversy has arisen with regard to per-operative evaluation of the exact length of the artificial cord. Moreover, when the implantation of several cords is required, for each of them it is required firstly to perform attachment to the papillary muscle and then measurement of the artificial chord approximately equal to the length of the normal chord and finally perform attachment to the edge of the cusp. Consequently, there exist major obstacles to the widespread use of this repair technique.

WO 96/40007 discloses a mitral prosthesis, whereas US 2004/143323 discloses a valve repair device having the features of the preamble of claim 1.

The object of the present mvention is to provide a device able to overcome the abovementioned problems.

The present invention therefore relates to a device for replacing the chordae tendineae of an atrioventricular valve according to claim 1.

In such a device, the cord elements are grouped together in a bundle inside a same device. The number of operations necessary for implanting the device is therefore reduced considerably.

Preferred embodiments of the invention are defined in the dependent claims.

Some preferred, but non-limiting embodiments of the invention will now be described with reference to the accompanying drawings in which:
- Figure 1 is a schematic and partially sectioned illustration of a human heart;
- Figure 2 is a view of a mitral valve from the left atrium;
- Figure 3 is a schematic illustration of a first embodiment of a replacement device according to the present invention;

- Figure 4 is a schematic illustration of a cusp of the mitral valve, connected by means of chordae tendineae to a papillary muscle; and
- Figure 5 is an illustration similar to that of Figure 4, which shows the device according to Figure 3 in an implanted condition;
- Figure 6 is a schematic illustration of an embodiment of a replacement device not according to the present invention;
- Figure 7 is a schematic representation of a further embodiment of a replacement device not according to the present invention;
- Figure 8 is a schematic representation of a further embodiment of a replacement device not according to the present invention; and
- Figure 9 is an illustration of a second embodiment of a replacement device according to the present invention.

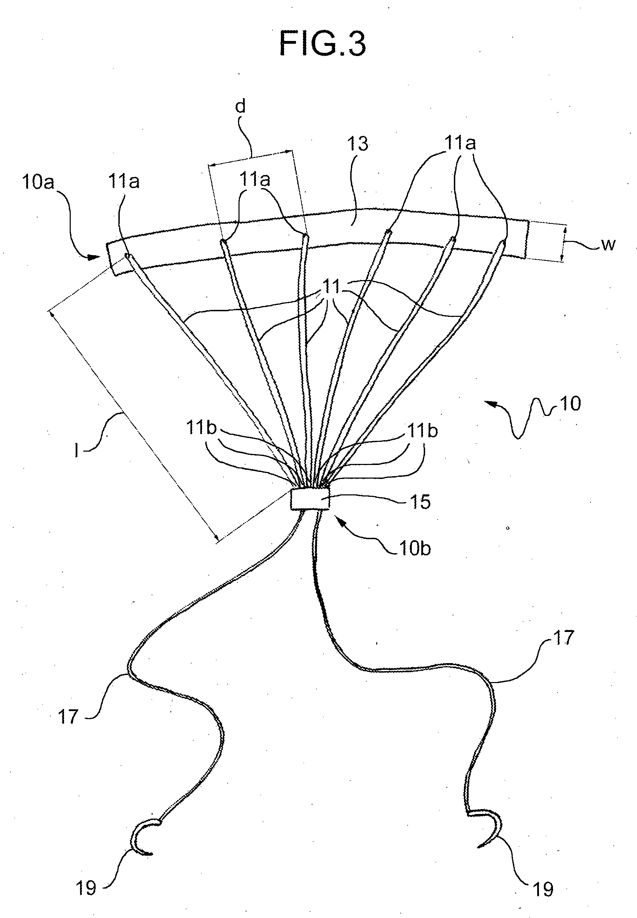

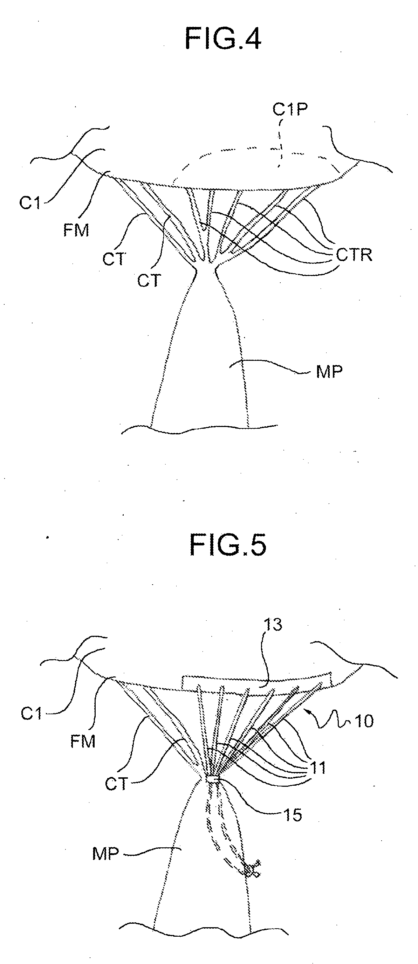

With reference to figures 3 to 5 and 9, these show some different embodiments of a device according to the invention, able to replace the chordae tendineae of an atrioventricular valve, in particular a mitral valve. As shown in Figure 5 and as will be clarified more fully below, the device according to the invention comprises a cuspidal end, which is able to be fixed along a portion of the free edge of a cusp of the atrioventricular valve, and an apical end, which is able to be fastened to an apex of a papillary muscle of the atrioventricular valve.

With reference to Figure 3, this now illustrates a first embodiment of a device according to the invention, denoted overall by 10. The device 10 comprises a cuspidal end, denoted by 10a, and an apical end, denoted by 10b. This device 10 also comprises a plurality of cord elements 11, which have a length approximately equal to the length of the natural chordae tendineae. The term "cord element" is understood as meaning a flexible and soft long element, such as, for example, a filament, a thread, a cord with a very small diameter, etc. The cord elements must be as flexible and soft as possible so that they do not impose undesirable restrictions on the movement of the cusps, but are nevertheless sufficiently strong to reduce the risk of breakages. In the embodiment shown, the device 10 comprises six cord elements 11. These cord elements have a length 1 of between about 22 mm and 26 mm. At the cuspidal end 10a of the device 10, each of the cords 11 is attached, by its end 11a, to a strip of flexible and soft material 13, which is able to simulate a portion of the free edge of a cusp of the atrioventricular valve. Alternatively, the cords 11 may be formed as one piece with the strip. The strip 13 may have a width w of between about 18 mm and 24 mm and have a distance d between the ends 11a of adjacent cords 11 equal to 3 mm. The measurements indicated here, however, are provided by way of example and are not to be regarded as limiting the invention.

At the apical end 10b of the device 10, the other ends 11b of the cords 11 are joined together and to a joining member 15, which is able to simulate the apex of a papillary muscle. The method of joining together the chords is not essential for the purposes of the invention. As an alternative to the mechanical joining member 15, it is possible to envisage, for example, that the cords 11 are joined together by means of bonding or are melted together or are knotted together so as to define a joining point where all the cords are united.

The joining member 15 has, extending from it, a pair of threads 17 provided at their free ends with needles 19 of the conventional type.

The mode of use of the device 10 according to the invention will now be described.

Figure 4 shows in schematic form the anterior cusp C1 of the mitral valve VM. In the vicinity of the free edge FM of the cusp C1, a bundle of chordae tendineae CT, CTR is connected to this cusp and terminate on the apex of the papillary muscle MP corresponding to the cusp C1. It is assumed that the cords indicated by CTR break. This situation causes prolapse of a portion C1P of the cusp C1, which is illustrated for explanatory purposes by means of a broken line.

Figure 5 shows the device 10 according to the invention in an implanted condition, such as to repair the prolapse of the cusp C1. The broken cords CTR have been removed beforehand. The strip 13 of the device 10 is fixed, for example by means of a continuous suture, to the edge FM of the prolapsed portion C1P of the cusp C1 of the mitral valve. At the other end of the cords 11, the joining member 15 of the cords 11 is fixed to the apex of the corresponding papillary muscle MP. The threads 17 are introduced inside the papillary muscle MP so as to pass through from the apex towards the base of the latter and are knotted together at their free ends emerging from the papillary muscle MP. The cords 11 of the device 10 replace the broken cords CTR.

In order to perform tethering of the device 10 to the papillary muscle MP the needles 19 were used. By means of these needles 19, with the use of surgical equipment of the conventional type, such as forceps, the threads 17 were introduced inside the papillary muscle MP and then passed out from this muscle. Once the needles 19 with the respective thread portions 17 were extracted from the papillary muscle MP, the needles 19 were cut off and the free ends of the threads 17 emerging from the papillary muscle MP were knotted together.

As can be understood, this embodiment of the device according to the invention not only reproduces the single chorda tendinea, but also the two attachment ends, namely the edge of the cusps and the apex of the papillary muscles.

If the length of the prolapsed segment is smaller than the length of the strip 13 of the device 10, it is possible to adapt the device 10 by simply cutting off one or more of the cords 11 and the excess strip portion 13.

Vice versa, if the length of the prolapsed segment is greater than the length of the strip 13 of the device 10, it is possible to use several devices arranged in a row along the same cusp.

If, instead, some cords of both the cusps C1 and C2, which however lead from the same papillary muscle MP, are broken, it is possible to use two devices 10 facing each other and connected on the one hand to the respective cusp and on the other hand to the papillary muscle concerned.

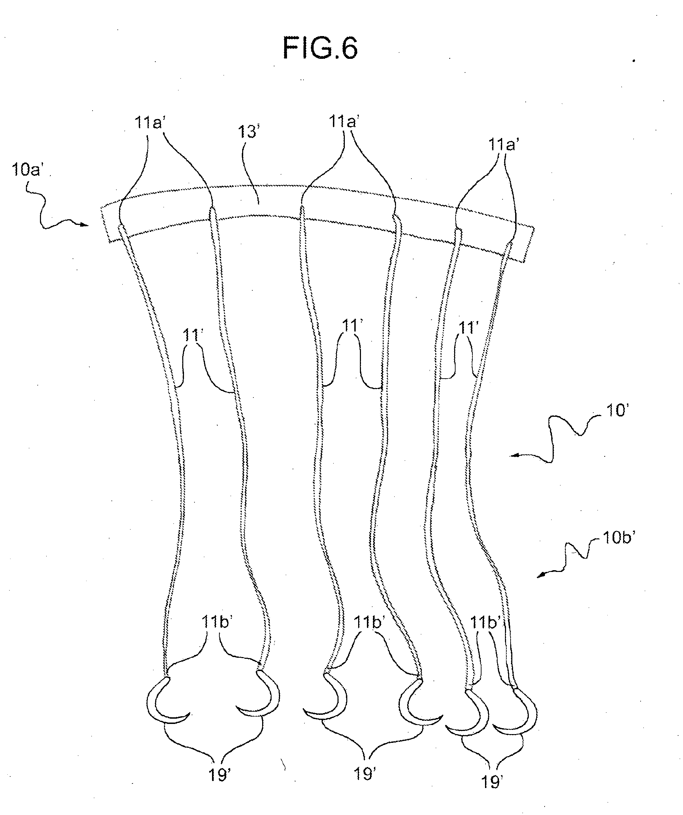

With reference to Figure 6, an example of a device not according to the invention, denoted overall by 10', is now described. The parts corresponding to those of the previous embodiment have been indicated with the same reference numbers, with the addition of an apostrophe, and will not be further described. The device 10' also comprises a cuspidal end, denoted by 10a', and an apical end, denoted by 10b'. This device 10' also comprises a plurality of cord elements 11'. At the cuspidal end 10a' of the device 10', the cord elements 11' are connected to a strip of flexible and soft material 13'. Differently from the first embodiment, at the apical end 10b' of the device 10' the cord elements 11' have ends 11b' which are free and not joined together. The free ends 11b' are provided with needles 19' of a type similar to that described with reference to the previous embodiment.

As can be understood, implantation of the device 10' of the example is slightly different from that of the device 10 of the first embodiment as regards the apical end of the device. In fact, here, it is necessary to fasten individually each cord element 11' to the apex of the papillary muscle, while in the previous embodiment a single operation for fastening all the cord elements 11 was sufficient.

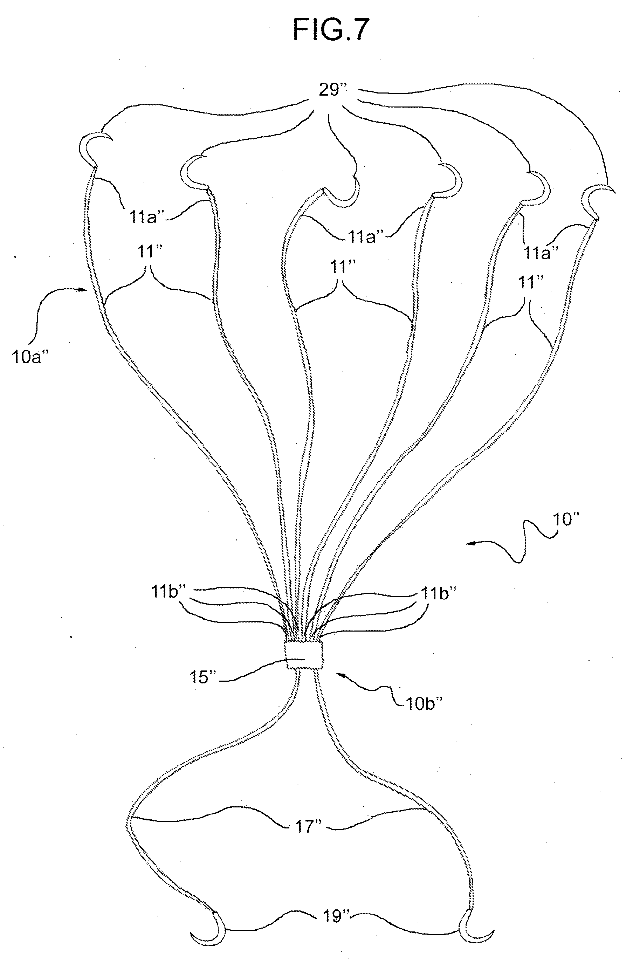

With reference to Figure 7, a further example of a device not according to the invention, denoted overall by 10", is now be described. The parts corresponding to those of the first embodiment have been indicated by the same reference numbers, with the addition of a double apostrophe, and will not be described further. The device 10" also comprises a cuspidal end, denoted by 10a", and an apical end, denoted by 10b". This device 10" also comprises a plurality of cord elements 11". At the apical end 10b" of the device 10", the cord elements 11" are joined together. Differently from the first embodiment, at the cuspidal end 10b" of the device 10" the cord elements 11" have ends 11a" which are free and not joined together. The free ends 11a" are provided with needles 29" of a type similar to those described above.

As can be understood, implantation of the device 10" of the further example is slightly different from that of the device 10 of the first embodiment as regards the cuspidal end of the device. In fact, here it is necessary to fasten individually each cord element 11" to the free edge of the valve cusp, while in the first embodiment a single operation for fastening all the cord elements 11 was sufficient.

Although the invention has been described with reference to the treatment of mitral prolapse, it is certainly not limited to this application, but may be used for all types of pathologies which involve the papillary muscles, chordae tendineae and valve cusps.

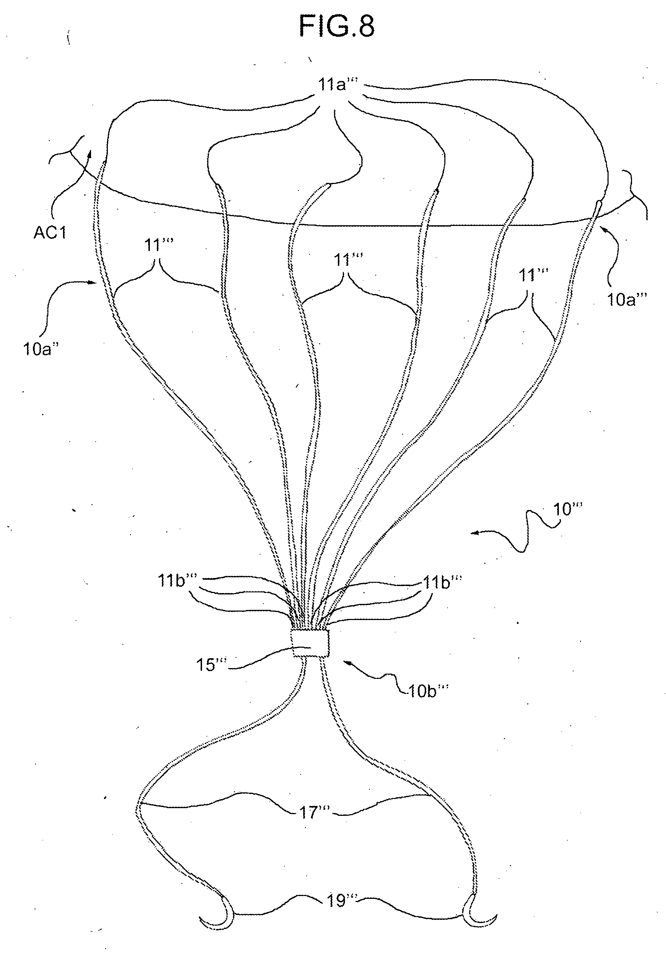

With reference to Figure 8, a further embodiment of a device not according to the invention, denoted overall by 10"', is described. The parts corresponding to those of the first embodiment have been indicated by the same reference numbers, with the addition of a triple apostrophe, and will not be further described. The device 10"' comprises also a cuspidal end, denoted by 10a"', and an apical end, denoted by 10b"'. This device 10"' furthermore comprises a plurality of cord elements 11"'. Differently from the first embodiment, the device 10"' forms part of an atrioventricular valve prosthesis of the conventional type. The cord elements 11"' are therefore connected to a movable part of the valve prosthesis intended to replace a cusp, denoted by AC1 in Figure 8. In the claims which follow, the term "cusp" may therefore be regarded as referring to both a cusp of a natural valve and to an artificial movable part of an atrioventricular prosthesis, and the term "valve" may refer both to a natural valve and to an atrioventricular valve prosthesis.

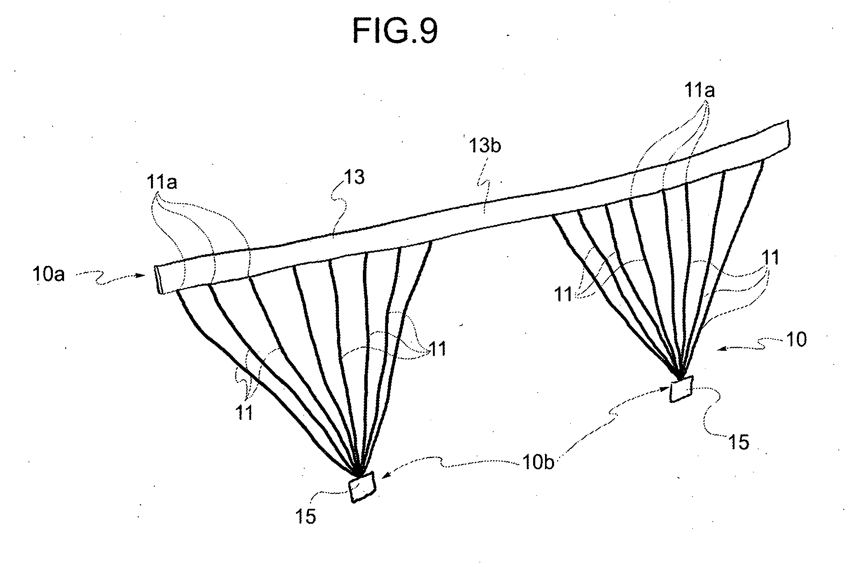

With reference to Figure 9, a second embodiment of a device according to the invention is now described. This embodiment is substantially the same as that of Fig. 3, and so the same reference numbers have been used. Therefore, the device 10 shown in Fig. 9 comprises a cuspidal end, denoted by 10a, and an apical end, denoted by 10b, and a plurality of cord elements 11, having a length approximately equal to the length of the natural chordae tendineae. At the cuspidal end 10a of the device 10, each of cords 11 is attached, by its end 11a, to a strip of flexible and soft material 13, which is able to simulate a portion of the free edge of a cusp of the atrioventricular valve. Alternatively, the cords 11 may be formed as one piece together with the strip.

The device according to Fig. 9 is different from that of Fig. 3 solely with regard to the configuration of the ends 11b of the cords at the apical end 10b.

In fact, at the apical end 10b of the device 10 according to Fig. 9, the ends 11b of a group of cords 11 are joined together, while the ends 11b of another group of cords 11 are joined together, but separate from those of the first group. At the apical end 10b of the device 10, the ends 11b of the cords 11 of each group are joined together and to a joining member 15, able to simulate the apex of a papillary muscle.

The device 10 according to Fig. 9 is also suitable for the treatment of ischemic mitral regurgitation. In this case there is no prolapse of the cusp, but retraction of the papillary muscle. For example, after a myocardial infarction, a papillary muscle retracts by 8 mm, exerting a pulling force on the chordae tendineae and on the corresponding edge of the cusp, resulting in an insufficiency. If the normal distance between the apex of the papillary muscle and the edge of the cusp is 24 mm, it would be possible to treat this case by implanting a device of 24 + 8 = 32 mm. The strip 13 of the device 10 is able to be fixed to the edge of the cusp of the mitral valve. At the other end of the cords 11, the device 10 according to Fig. 9 is able to be fixed to the apex of two separate papillary muscles, for example by means of suture needles. Advantageously, the centre 13b of the strip 13 corresponds exactly to the centre of the anterior or posterior leaflet (centre of A2 or P2). This is important because the cords are distributed equally between the two papillary muscles.

The device according to the invention, in the various embodiments described above, may be made in different sizes (length, distance between the apex of the papillary muscle and edge of cusps). This represents a major simplification since, once the distance from the apex of the papillary muscle to the plane of normal coaptation of the leaflets has been determined, the surgeon needs only choose a device of suitable size. This distance may be calculated by means of echocardiography, nuclear magnetic resonance or during repair of the valve.

Obviously, without modifying the principle of the invention, the constructional details and the embodiments may be greatly varied with respect to what described and illustrated, without thereby departing from the scope of the invention.

## Claims

1. Device (10) for replacing the chordae tendineae (CT) of an atrioventricular valve, wherein said device comprises a cuspidal end (10a), an apical end (10b), and a plurality of cord elements (11) extending between said cuspidal end and said apical end, **characterized in that** at said cuspidal end (10a) the cord elements (11) are connected together by a strip part (13) so as to be spaced from each other, said strip part (13) being able to be fixed along a portion (C1P) of the free edge (FM) of a cusp (C1) of said atrioventricular valve, and **in that** at said apical end (10b) the cord elements (11) are joined together at a joining point (15), and fastening means (17, 19) are provided at said joining point in order to fasten said plurality of cord elements to an apex of a papillary muscle (MP) of said atrioventricular valve.

2. Device according to Claim 1, in which, at said joining point, said cord elements are joined together by means of bonding.

3. Device according to Claim 1, in which, at said joining point, said cord elements are melted together.

4. Device according to Claim 1, in which, at said joining point, said cord elements are knotted together.

5. Device according to one of Claims 1 to 4, in which, at said joining point, said cord elements are joined together by means of a joining member (15).

6. Device according to one of Claims 1 to 5, in which said fastening means comprise a pair of threads (17) able to be introduced inside said papillary muscle so as to pass through from the apex to the base of the latter, so as to be able to be knotted together at their free ends emerging from said papillary muscle.

7. Device according to Claim 6, in which said threads are, at the respective free ends, provided with needles (19) for introducing said threads inside said papillary muscle.

8. Device according to Claim 1, in which said strip part is formed as one piece with said cord elements.

9. Device according to Claim 1, in which said cord elements are attached to said strip part.

## Patentansprüche

1. Vorrichtung (10) zum Austauschen der Sehnenfäden (CT) einer Atrioventlikularklappe, wobei die Vorrichtung ein kuspidales Ende (10a), ein apikales Ende (10b) und eine Vielzahl von sich zwischen dem kuspidalen Ende und dem apikalen Ende erstreckenden Schnurelementen (11) umfasst,
**dadurch gekennzeichnet,**
**dass** die Schnurelemente (11) an dem kuspidalen Ende (10a) mittels eines Streifenteils (13) miteinander verbunden sind, sodass sie mit Abstand zu einander angeordnet sind, wobei das Streifenteil (13) geeignet ist, entlang eines Bereichs (C1P) des freien Randes (FM) eines Herzklappensegels (C1) der Atrioventlikularklappe fixiert zu werden, und dass die Schnurelemente (11) an dem apikalen Ende (10b) in einem Verbindungspunkt (15) miteinander verbunden sind, und Befestigungsmittel (17, 19) an diesem Verbindungspunkt vorgesehen sind, um die Vielzahl von Schnurelementen an einem Apex eines Papillarmuskels (MP) der Atrioventrikularklappe zu befestigen.

2. Vorrichtung nach Anspruch 1,
**dadurch gekennzeichnet,**
**dass** die Schnurelemente in dem Verbindungspunkt mittels Verklebung miteinander verbunden sind.

3. Vorrichtung nach Anspruch 1,
**dadurch gekennzeichnet,**
**dass** die Schnurelemente in dem Verbindungspunkt miteinander verschmolzen sind.

4. Vorrichtung nach Anspruch 1,
**dadurch gekennzeichnet,**
**dass** die Schnurelemente in dem Verbindungspunkt miteinander verknotet sind.

5. Vorrichtung nach einem der Ansprüche 1 bis 4,
**dadurch gekennzeichnet,**
**dass** die Schnurelemente in dem Verbindungspunkt mittels eines Verbindungselementes (15) miteinander verbunden sind.

6. Vorrichtung nach einem der Ansprüche 1 bis 5,
**dadurch gekennzeichnet,**
**dass** die Befestigungsmittel ein Paar Fäden (17) umfasst, die geeignet sind, in das Innere des Papillarmuskels eingeführt zu werden, sodass das Paar Fäden von dem Apex zu der Basis des Apex verläuft, sodass die Möglichkeit besteht, die freien Enden der Fäden, die von dem Papillarmuskel ausgehen, miteinander zu verknoten.

7. Vorrichtung nach Anspruch 6,
**dadurch gekennzeichnet,**
**dass** die Fäden an ihren jeweiligen freien Enden mit Nadeln (19) zur Einführung der Fäden in den Papillarmuskel ausgestattet sind.

8. Vorrichtung nach Anspruch 1,
**dadurch gekennzeichnet,**
**dass** das Streifenteil einstückig mit den Schnurelementen ausgebildet ist.

9. Vorrichtung nach Anspruch 1,
**dadurch gekennzeichnet,**
**dass** die Schnurelemente an dem Streifenteil befestigt sind.

## Revendications

1. Dispositif (10) pour remplacer les cordes tendineuses (CT) d'une valve atrioventriculaire, dans lequel ledit dispositif comprend une extrémité cuspide (10a), une extrémité apicale (10b), et une pluralité d'éléments de corde (11) s'étendant entre ladite extrémité cuspide et ladite extrémité apicale, **caractérisé en ce que**, au niveau de ladite extrémité cuspide (10a), les éléments de corde (11) sont reliés ensemble par une partie de bande (13) de manière à être espacés les uns des autres, ladite partie de bande (13) pouvant être fixée le long d'une partie (C1P) du bord libre (FM) d'une valvule (C1) de ladite valve atrioventriculaire, et **en ce que**, au niveau de ladite extrémité apicale (10b), les éléments de corde (11) sont reliés ensemble au niveau d'un point de jonction (15), et des moyens de fixation (17, 19) sont prévus au niveau dudit point de jonction afin de fixer ladite pluralité d'éléments de corde à un apex d'un muscle papillaire (MP) de ladite valve atrioventriculaire.

2. Dispositif selon la revendication 1, dans lequel, au niveau dudit point de jonction, lesdits éléments de corde sont reliés ensemble par liaison.

3. Dispositif selon la revendication 1, dans lequel, au niveau dudit point de jonction, lesdits éléments de corde sont fusionnés ensemble.

4. Dispositif selon la revendication 1, dans lequel, au niveau dudit point de jonction, lesdits éléments de corde sont noués ensemble.

5. Dispositif selon l'une des revendications 1 à 4, dans lequel, au niveau dudit point de jonction, lesdits éléments de corde sont reliés ensemble par un élément de jonction (15).

6. Dispositif selon l'une des revendications 1 à 5, dans lequel lesdits moyens de fixation comprennent une paire de fils (17) pouvant être introduits à l'intérieur dudit muscle papillaire de manière à traverser depuis l'apex jusqu'à la base de ce dernier, en pouvant ainsi être noués ensemble au niveau de leurs extrémités libres sortant dudit muscle papillaire.

7. Dispositif selon la revendication 6, dans lequel lesdits fils sont, au niveau de leurs extrémités libres respectives, munis d'aiguilles (19) pour introduire lesdits fils (17) à l'intérieur dudit muscle papillaire.

8. Dispositif selon la revendication 1, dans lequel ladite partie de bande est faite d'une seule pièce avec lesdits éléments de corde.

9. Dispositif selon la revendication 1, dans lequel lesdits éléments de corde sont attachés à ladite partie de bande.
